# EUROPEAN PATENT APPLICATION

(11) **EP 0 570 239 A2**
(43) Date of publication of application: **18.11.1993**
(21) Application number: 93303738.4
(22) Date of filing: 14.05.1993
(51) Int. Cl.: G01K 7/06

(54) **Thermocouple**

(30) Priority: 14.05.1992 GB 9210377
(71) Applicant: LUCAS INDUSTRIES PUBLIC LIMITED COMPANY, Solihull, West Midlands B91 3TX (GB)
(72) Inventor: Cross, David Henry, Acocks Green, Birmingham, B27 6HB (GB); Taylor, Alfred James, Solihull, West Midlands, B92 9PF (GB)
(74) Representative: Lawrence, John

(57) **Abstract**

A thermocouple (1) being one integral member has layers of conducting and insulating materials (3, 4, 6). The thermocouple has a single wire (2), coated with an insulating layer (4). The insulating layer is itself coated with a conducting layer (3) which is in contact with the wire (2) in the vicinity of a tip region (5) of the thermocouple, the tip region (5) forming the thermocouple junction region. The thermocouple is provided with an overall protective coating (6) of hard wearing material.

## Description

This invention relates to thermocouples, methods of making them, and to apparatus incorporating them.

Thermocouples have been known for many years and there are many known types having different structures. An aim of this invention is to provide a new thermocouple. Another aim is to provide a new way of making a thermocouple.

According to a first aspect we provide a thermocouple comprising a carrier member of a first material which carries a thin layer or film of a second material, the carrier member and the film contacting each other at a thermocouple junction region.

By applying a thin film to a carrier member we have found that our thermocouple can be made to have a fast response to temperature changes. Furthermore, this also allows us to make a compact thermocouple. When the carrier member is a wire we can provide a one-wire thermocouple sensor. We can improve the response time of the thermocouple by using thinner wires as carrier members.

Preferably the carrier member comprises a body, which preferably contributes at least in part to the mechanical strength of the thermocouple, at least in the region of the thermocouple junction. The carrier member is preferably a wire or the like.

The carrier member is preferably elongate and preferably has the thermocouple junction region at one end; at its tip. The tip of the carrier member and/or the thermocouple preferably has a convergent cross section and preferably ends in a point, or a rounded point.

The film preferably surrounds or substantially fully coats the carrier member, but it could alternatively not extend completely around the circumferential extent of the carrier member; for example it may comprise an elongate strip. The film preferably completely encloses the tip of the member at the thermocouple junction.

The thermocouple preferably has a layer or coating of insulating material between the carrier member and film of second material, the insulating coating not being present between the carrier member and the film at the thermocouple junction. The insulating coating preferably extends over a substantial part of the elongate length of the carrier member and/or film, or at least over a substantial part of their overlapped length.

The film of second material is preferably protected from mechanical trauma and environmental hazards by a protective insulating member overlying it. The protective member is preferably a layer or coating. The protective layer may comprise ceramic material, or a mineral such as quartz.

A protective layer makes the thermocouple more robust. However, it should be noted that the basic structure of coating a central carrier member with the second electrode of the thermocouple is in itself an intrinsically robust structure.

An anti-diffusion barrier may be provided between the carrier member and the film of second material at a region spaced from the thermocouple junction, and/or between the film and the protective member.

According to a second aspect the invention consists in temperature sensing apparatus, or a temperature sensing system, incorporating a thermocouple in accordance with the first aspect of the invention.

The system may comprise a fuel injection system, the thermocouple providing an indication of the temperature in the engine, and/or in a cylinder of the engine.

The system may comprise a control system for applying heat.

According to a third aspect the invention consists in a method of manufacturing a thermocouple comprising the steps of taking a longitudinally extending carrier member of one material, applying a film or thin layer of a different material so that a thermocouple junction is formed where the two materials meet, and insulating the film from the carrier member at regions of the longitudinal length of the carrier member other than the thermocouple junction.

Preferably the thermocouple junction is arranged to be at the tip of the carrier member.

Preferably the film is applied so as to enclose the tip of the carrier member.

Preferably the method further comprises the step of applying an insulating layer to the carrier member before the film is applied.

The method also preferably comprises forming a pointed or rounded tip to the carrier member and/or the thermocouple, preferably after the insulating layer has been applied, and most preferably before the film is applied.

Alternatively the tip could be left free of insulating material when the insulating layer is applied and the film applied to contact the other thermocouple material, without any insulating material removing operation. This is preferably achieved by suitable masking techniques during deposition of successive layers.

Preferably an anti-diffusion layer is applied to the carrier member before the film is applied. Preferably the anti-diffusion layer is removed from the tip of the carrier member as the tip is rounded or pointed so that when the film is applied the film contacts the carrier member directly at the tip. Alternatively the anti-diffusion layer may be applied so as not to overlie the tip, leaving it free for contact with the other thermocouple material.

When the insulating layer and/or anti-diffusion layer is applied so as not to cover the tip of the carrier member the tip of the carrier member may have a rounded shape, but there may be no need to round the tip: it could conceivably simply be the end of a piece of wire.

According to a fourth aspect the invention consists in a method of sensing the temperature of a region comprising using a thermocouple probe in accordance with the first or third aspects of the invention.

An embodiment of the invention will now be described by way of example only with reference to the accompanying drawings of which:-
Figure 1 show schematically a thermocouple in accordance with the invention;
Figures 2A to 2G show schematically stages in the production of another thermocouple;
Figures 3A and 3B show the incorporation of the thermocouple of Figure 1 or Figures 2G into a fuel injector for a diesel engine.

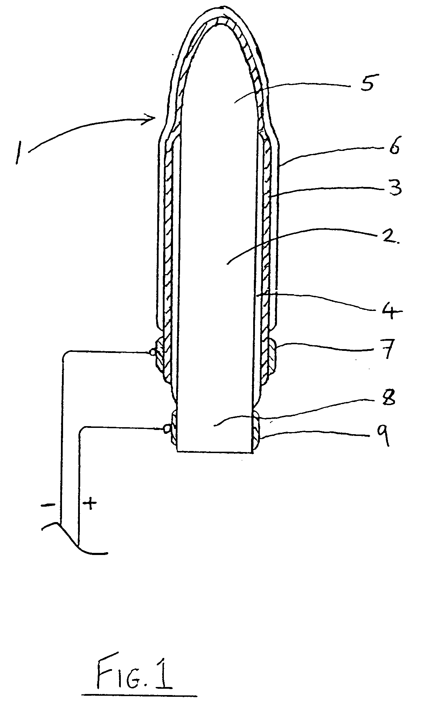

Figure 1 shows a one-wire fast response thin film thermocouple probe 1 which comprises a wire 2 of nickel/chromium alloy (chromel) which forms one electrode of the thermocouple, and a thin film 3 of nickel/aluminium alloy (alumel) which forms the second electrode of the thermocouple and which is supported on the wire 2. An insulating coating 4, for example of quartz, extends between the film 3 and the wire 2 for most of the longitudinal length of the body of the probe 1, but does not exist in the forwardmost tip region 5 where the film and wire contact each other to form a thermocouple junction. A quartz protective layer 6 covers most of the body of the probe 1, including the tip 5. A solder contact ring 7 for the film 3 surrounds the probe towards the rear of the film layer, which film layer stops short of a rearward position 8 of the wire 2. The insulating coating 4 also stops short of the rearward portion 8. A second solder contact ring 9 for the wire 2 is provided at the rearward portion 8. The solder rings 7 and 9 are made of a layer of nichrome alloy, then a layer of palladium, then a layer of gold.

The wire 2 has a diameter of about 6 thousands of an inch ( 150µm), the insulating layer 4 a thickness of about 2µm, the film 3 a thickness of about 0.5µm, and the protective coating 6 a thickness of about 1.5µm. The longitudinal length of the tip 5 where the wire and film are in direct contact is about 0.5µm. The drawings are not to scale.

The thermocouple probe 1 is used in any suitable temperature sensor, with the solder rings 9 and 7 being connected to the positive and negative input terminals of the sensor's electronics. The tip 5 experiences a different temperature than that of the region of the two solder contacts and a thermocouple e.m.f. is produced indicative of this temperature difference. It may be desirable to have the two solder rings close together.

The tip 5 of the probe is mechanically worked to round it off so that the thin film 3 adheres to the rounded surface better than it would to a surface with sharp points, and so that when the film is deposited by thin film processing, for example magnetron sputtering physical vapour deposition (PVD) techniques, the film can be deposited properly. There may be other ways of providing the desired shape of the tip of the probe.

The choice of materials for the wire and film depend upon the intended use of the probe, as do the dimensions of the wire and film. When certain materials are used at high temperatures, or when the probe is made at high temperatures, it may be necessary to incorporate diffusion barriers. For example in the embodiment of Figure 1 the aluminium in the alumel film may diffuse into the quartz at temperatures above about 250°C.

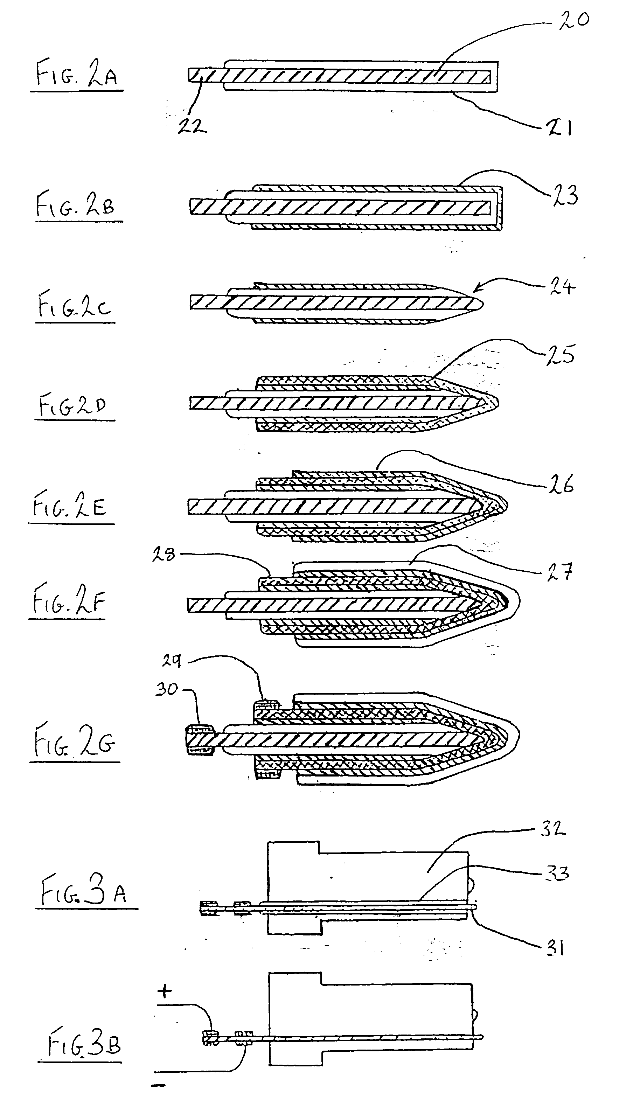

Figures 2A to 2G show stages in the manufacture of a K-type thermocouple similar to that shown in Figure 1, but with anti-diffusion barriers.

A chromel wire, strip, or the like, 20 is cleaned and then coated using PVD with a quartz insulating layer 21 over its entire length, except for an end 22 which is to be used for a solder connection. This is shown in Figure 2A.

A nichrome anti-diffusion layer 23 is then deposited over the insulating layer 21, as shown in Figure 2B.

A tip 24 of the wire 20 to be used for the thermocouple junction is then polished as shown in Figure 2C. This serves both to remove the insulating layer 21 and to form a rounded dome-like profile at the end of the wire. This rounded profile allows complete coverage around the corners of the wire tip by a thin film conducting layer 25.

Figure 2D shows the deposition of the thin film conducting layer 25 of alumel over the insulating layer 21 and the exposed tip 24. Thus the thermocouple junction is completed only at the wire's rounded tip.

Figure 2E shows the deposition of a further nichrome anti-diffusion layer 26 in order to prevent diffusion of the alumel film into the next, outer, layer of quartz to be deposited.

A protective layer 27 of quartz then is deposited on the thin film conductor 25 both electrically to insulate and environmentally protect the thermocouple device. An end 28 of the thin film 15 spaced away from the thermocouple tip 24 is, however, left exposed to facilitate solder connections for external wires. This is shown in Figure 2F.

Figure 2G shows the deposition of solder contact pads 29 and 30 of layers of nichrome, palladium, and gold on the exposed end 22 of the wire 20 and the exposed end 28 of the thin film conductor 25.

The "one wire" thermocouple (referenced 31) is then mounted in an appropriate body, for example, an internal combustion engine component such as a diesel fuel injector 32, shown in Figure 3A. The "one wire" thermocouple is bonded to the injector, for example by forming glass to metal seals, or by using resins or similar bonding agents. The glass/metal seal, or the bonding agent, is referenced as 33 in Figure 3A.

The "one wire" thermocouple 31 is then connected by soldering to external connections. The external connections may be encapsulated, for example in a resin. These connections then take the thermocouple output to appropriate temperature instrumentation. Sealing the solder contacts in resin provides a sealed unit and reduces the risk of the connection wires moving and breaking off.

An alternative way of making a "one-wire" thermocouple is to arrive at the arrangement of Figure 2C without the need to remove material from the tip of the component. Known masking techniques enable us to control the deposition position of the insulating layer 21 such that it can be applied so that it does not cover the tip of the wire 20, leaving the tip of the wire available to form a thermocouple junction. Similarly, the anti-diffusion layer 23 can be applied directly into the position shown in Figure 2C using controlled deposition of material. The manufacture of the thermocouple then follows the arrangement of Figures 2D to 2G.

In the above controlled disposition position techniques we prefer to round the tip of the wire 20 as the first step, before deposition of layers 21 and 23. Alternatively the tip of the wire need not be rounded. We also prefer to set back the position of the end of the anti-diffusion layer 23 from the end of the insulating layer 21 (as shown in Figure 2C), but this may not be essential.

The "one wire" thermocouple can be used to monitor rapid temperature changes because its response time is good. The "one wire" thermocouple has applications in the automotive industry, for example to monitor the start and end of combustion in internal combustion engines. This information can be used in fuel injection systems and engine management systems.

The "one wire" thermocouple could also have possible uses for control in laser cutting and processing, for example of semiconductors; in medical treatments and diagnostics with lasers in the UV, visible or IR; or in other applications where its small size, and especially its small sensing tip, allows it to detect the temperature of a very small area. This may facilitate its use in guidance or control systems. Its small size also allows its use in endoscopic applications. The fast response of the thermocouple allows its use in situations where a fast response is necessary, such as fuel temperature measurement in aerospace applications (eg temperature of fuel in the wings of an aircraft).

## Claims

1. A thermocouple (1) comprising a carrier member (2) of a first material carrying a thin layer or film (3) of a second material, the carrier member and the film contacting each other at a thermocouple junction region.

2. A thermocouple (1) according to claim 1 characterised in that the carrier member (2) is a wire or the like.

3. A thermocouple (1) according to claim 1 or claim 2 characterised in that the thermocouple junction region is provided at one end (5) of the carrier member.

4. A thermocouple (1) according to claim 3 characterised in that said one end (5) of the carrier member has a convergent cross section.

5. A thermocouple (1) according to any preceding claim characterised in that it has a layer or coating of insulating material (4) between the carrier member (2) and film (3) of second material.

6. A thermocouple (1) according to any preceding claim characterised in that the film (3) of second material is protected from mechanical trauma and environmental hazards by a protective insulating member (6) overlying it.

7. A thermocouple according to any preceding claim characterised in that an anti-diffusion barrier is provided between the carrier member (2) and the film (3) of second material and/or between the film and the protective member (6).

8. A method of manufacturing a thermocouple comprising the steps of taking a longitudinally extending carrier member (2) of one material, applying a film or thin layer of a different material (3) so that a thermocouple junction is formed where the two materials meet, and insulating the film from the carrier member at regions of the longitudinal length of the carrier member other than the thermocouple junction.

9. A method according claim 8 characterised in that the method further comprises the step of forming a pointed or rounded tip (5) to the carrier member (2) and/or the thermocouple (1).

10. A method according to claim 9 characterised in that the tip (5) is rounded after an insulating layer (3) has been applied.

11. A method according to any one of claims 8 to 10 characterised in that the method further comprises the steps of applying an anti-diffusion layer to the carrier member (2) before the film (3) is applied and removing the anti-diffusion layer from the tip (5) of the carrier member (2) as the tip (5) is rounded or pointed such that the film (3) directly contacts the carrier member (2) at the tip (5).

12. A method according to claim 8 or claim 9 characterised in that the insulating material (21) is applied to the carrier member (2) in such a manner that it does not overlie a region of the carrier member (2), leaving that region available to contact said film or thin layer (25) of a different material when that film or layer (25) is applied.
